# EUROPEAN PATENT APPLICATION

(11) **EP 3 922 233 A1**
(43) Date of publication of application: **15.12.2021**
(21) Application number: 20753251.6
(22) Date of filing: 28.01.2020
(51) Int. Cl.: A61J 3/00

(54) **MEDICAL DRUG VERIFICATION DEVICE, MEDICAL DRUG VERIFICATION METHOD, AND MEDICAL DRUG VERIFICATION SYSTEM**

(30) Priority: 07.02.2019 JP 2019020703
(71) Applicant: FUJIFILM Toyama Chemical Co., Ltd., Chuo-ku Tokyo 104-0031 (JP)
(72) Inventor: YOKOUCHI, Koji, Tokyo 104-0031 (JP)
(74) Representative: Dewhurst, Joseph Michael
(86) International application number: PCT/JP2020/002947
(87) International publication number: WO 2020/162263

(57) **Abstract**

The present invention has an object to provide a medical drug verification device, a medical drug verification method, and a medical drug verification system that can improve robustness in matching information on medical drugs. A medical drug verification device according to a first aspect of the present invention includes an exterior information acquiring unit configured to acquire exterior information on a medical drug, a matching unit configured to perform matching between matching target information based on the acquired exterior information and a reference information group of medical drugs stored in a storage device, a display control configured to cause a display device to display, based on the degree of the matching, a plurality of candidates of reference information for a medical drug which is contained in the reference information group and corresponds to the exterior information, an accepting configured to accept selection of the reference information contained in the plurality of candidates, and a reflecting configured to reflect the exterior information and/or the matching target information corresponding to the selected reference information in the stored reference information group, wherein the reflecting unit performs the reflection when the degree of the matching for the selected reference information does not satisfy a predetermined criterion.

## Description

### BACKGROUND OF THE INVENTION

### 1. Field of the Invention

The present invention relates to a medical drug verification device, a medical drug verification method, and a medical drug verification system that perform matching of information on medical drugs.

### 2. Description of the Related Art

Audit of medical drugs and identification of medical drugs brought by patients are performed at hospital facilities, pharmacies and the like. If these works are performed by visual check, the workload on pharmacists and the like is heavy. Therefore, a technique for supporting the audit or the identification has been developed. For example, it is known that a computer performs audit support and identification support by matching images of medical drugs, and Patent Literature 1 can be cited as an example of such a technique. Patent Literature 1 describes a medical drug identification system in which images obtained by imaging a medical drug with a plurality of cameras and a plurality of light sources are used and pattern-matched with template images to identify the medical drug.

### Citation List

Patent Literature 1: WO2015/152225

### SUMMARY OF THE INVENTION

Matching in auditing or identifying medical drugs is affected, for example, by matching conditions such as individual differences in medical drugs and characteristics of master images as described below.

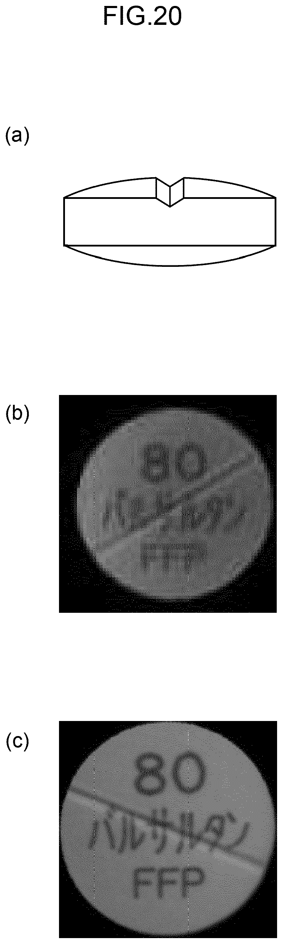

Figure 20 is a diagram showing examples of a bisection line and a printed character of a tablet. Portion (a) of Figure 20 is a side view of the tablet, and as can be seen from the portion, the tablet has a bisection line on one side thereof. Portion (b) of Figure 20 is an example showing the relationship between the bisection line and the printed character, and the bisection line is appended in a direction from the lower left side to the upper right side of the portion (b). Portion (c) of Figure 20 is another example showing the relationship between the bisection line and the printed character for the same medical drug, and the bisection line is appended in a direction from the upper left side to the lower right side of the portion (c). As described above, even in the case of the same medical drug, the relationship between the bisection line and the printed character may differ among individuals.

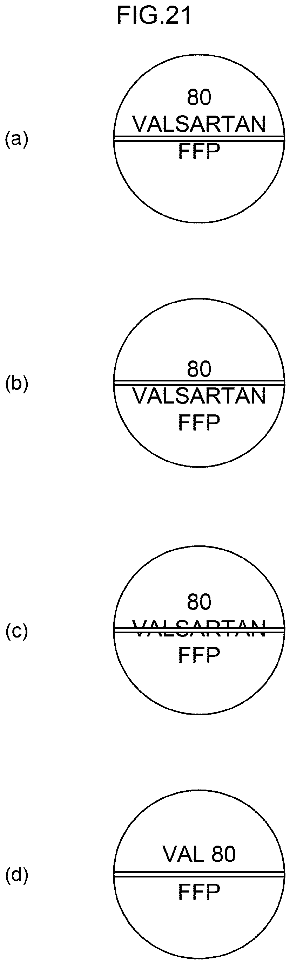

Figure 21 is another diagram showing the relationship between the bisection line and the printed character. Each of portions (a) and (b) of Figure 21 shows an example in which there are two rows of printed characters on an upper or lower side of the bisection line, and portion (c) shows an example in which some of the printed characters are erased and unidentified by the bisection line. Further, there is a case where the printed character is changed even in the case of the same medical drug as shown in portion (d) of Figure 21. The individual differences of the medical drugs shown in Figures 20 and 21 may also occur in the case of an embossment. Further, the same problem may occur not only for matching target medical drugs, but also for master images.

However, conventional techniques such as Patent Literature 1 do not fully consider these circumstances, and have low robustness in matching information on medical drugs.

The present invention has been made in view of such circumstances, and has an object to provide a medical drug verification device, a medical drug verification method, and a medical drug verification system that can enhance robustness in matching information on medical drugs.

In order to attain the above object, a medical drug verification device according to a first aspect of the present invention includes an exterior information acquiring unit configured to acquire exterior information on a medical drug, a matching unit configured to perform matching between matching target information based on the acquired exterior information and a reference information group of medical drugs stored in a storage device, a display control unit configured to cause a display device to display, based on a degree of the matching, a plurality of candidates of reference information for a medical drug which is contained in the reference information group and corresponds to the exterior information, an accepting unit configured to accept selection of the reference information contained in the plurality of candidates, and a reflecting unit configured to reflect the exterior information and/or the matching target information corresponding to the selected reference information in the stored reference information group, wherein the reflecting unit performs the reflection when the degree of the matching for the selected reference information does not satisfy a predetermined criterion.

In the first aspect, when the degree of matching of the selected reference information does not satisfy the predetermined criterion (for example, when it is difficult to perform definitive matching), the candidates of the reference information are displayed on the display device, so that a user can select correct reference information (reference information corresponding to the acquired exterior information). The exterior information and/or the matching target information corresponding to the selected reference information is reflected in the reference information group. In this way, when the degree of matching does not satisfy the criterion, the reference information group is subjected to the reflection based on the selected reference information, so that appropriate reference information (group) can be used, and it is possible to improve the robustness in matching information on medical drugs.

In the first aspect, matching may be performed by using the acquired exterior information, or matching may be performed by using the matching target information based on the acquired exterior information (information generated by processing the acquired exterior information). For example, extraction of a medical drug region, and extraction or emphasis of an embossment/or a printed character may be performed as the processing to be performed when the matching target information is used. Such processing can be performed, for example, based on a plurality of images imaged while changing the illumination condition. Further, a value of degree of coincidence, degree of similarity, degree of reliability, or correlation of information can be used as "degree of matching".

In the first aspect and other aspects described later, information capable of specifying the shape, dimension, embossment and/or printed character, color, etc., of a medical drug can be referred to as "exterior information". Further, information serving as a reference in matching for the shape, dimension, embossment and/or printed character, color, etc., of a medical drug can be referred to as "reference information", and an aggregation of these reference information pieces can be referred to as "reference information group". A two-dimensional image of the medical drug can be used as these information pieces, but a three-dimensional image or other measurement data may be used instead of the two-dimensional image.

According to a medical drug verification device of a second aspect, in the first aspect, the reflecting unit additionally stores, in the storage device, the exterior information and/or the matching target information corresponding to the selected reference information as new reference information for a medical drug designated by the exterior information, thereby performing the reflection. The second aspect defines one aspect of reflection, and when the degree of matching does not satisfy a predetermined criterion, reference information is additionally stored (reference information increases). A plurality of reference information pieces may be stored for one type of medical drug.

According to a medical drug verification device of a third aspect, in the first aspect, the reflecting unit generates new reference information based on the exterior information and/or the matching target information corresponding to the selected reference information and the stored reference information, and updates the stored reference information with the generated reference information, thereby performing the reflection. The third aspect defines one aspect of reflection, and when the degree of matching does not satisfy a predetermined criterion, the reference information is updated with new reference information. Therefore, the quantity of reference information for each medical drug is maintained. Note that the new reference information can be generated by weighting and addition of a plurality of information pieces or the like, but a generating method is not limited to such an aspect.

According to a medical drug verification device of a fourth aspect, in any one of the first to third aspects, when the degree for the selected reference information does not satisfy a predetermined reference value, the reflecting unit determines that the degree does not satisfy the criterion, and performs the reflection. The fourth aspect defines one aspect of "when the criterion is not satisfied", and values of degree of coincidence, degree of similarity, degree of reliability, correlation, etc., may be used as "degree (of matching)" as described above in the first aspect.

According to a medical drug verification device of a fifth aspect, in any one of the first to fourth aspects, when ranking determined by the degree for the selected reference information is lower than predetermined ranking, the reflecting unit determines that the degree does not satisfy the criterion and performs the reflection. The fifth aspect defines one aspect of "when the criterion is not satisfied", for example, "the reflection is performed when the ranking determined by the degree of matching is lower than first ranking (is second ranking or less), it is reflected" may be set.

According to a medical drug verification device of a sixth aspect, in any one of the first to fifth aspects, the medical drug verification device further includes an information processing unit configured to subject the exterior information to processing for emphasizing an embossment and/or a printed character of a medical drug, and the matching unit performs the matching by using the exterior information which has been subjected to the processing. By performing matching using the information subjected to the emphasis processing in the sixth aspect, the accuracy of matching can be improved.

According to a medical drug verification device of a seventh aspect, in any one of the first to sixth aspects, the reflecting unit stores the reference information in the storage unit in association with timing information indicating a timing of the reflection. For example, the date and time of reflection can be used as "information indicating the timing of reflection", but other information may be used.

According to a medical drug verification device of an eighth aspect, in the seventh aspect, the display control unit causes the display device to display the reference information and the timing information in association with each other. According to the eighth aspect, the user can visually recognize the timing at which the reflection is performed and the reference information at that timing.

According to a medical drug verification device of a ninth aspect, in any one of the first to eighth aspects, the medical drug verification device further includes an imaging unit configured to image the medical drug, wherein the exterior information acquiring unit acquires an image imaged by the imaging unit. In the ninth aspect, it is preferable to image the medical drug in a plurality of directions by a measure of using a plurality of imaging devices or a movable imaging device, or a device for changing the position and posture of medical drugs or the like. Further, in the ninth aspect, an image imaged by the imaging unit or an image obtained by subjecting an imaged image to image processing may be used as "reference information" or "reference information group", whereby highly accurate matching can be performed by matching an imaging condition between a matching target medical drug and reference information.

According to a medical drug verification device of a tenth aspect, in any one of the first to ninth aspects, the medical drug verification device further includes a light source configured to illuminate the medical drug. The medical drug verification device according to the tenth aspect may include a plurality of light sources or a movable light source, or may be configured so that the illumination direction can be switched according to the imaging direction.

According to a medical drug verification device of an eleventh aspect, in any one of the first to tenth aspects, the medical drug verification device further includes a related information acquiring unit configured to acquire related information on a medical drug, wherein the matching unit performs the matching by using the reference information for a medical drug specified by the related information. In the eleventh aspect, data obtained by reading information described in a prescription, data which is input and/or edited by a user such as a doctor based on the information described in the prescription, data described on a notebook such as "medicine notebook", etc., may be used as "related information".

According to a medical drug verification of a twelfth aspect, in any one of the first to eleventh aspects, the exterior information, the reference information, and the reference information group include at least one of two-dimensional or three-dimensional shape data and/or exterior data of a medical drug, and a two-dimensional image or three-dimensional image of a medical drug. The twelfth aspect defines one aspect of the exterior information, the reference information, and the reference information group. In the twelfth aspect, it is preferable that the exterior information, the reference information, and the reference information group include the same kind of information ("if the exterior information includes a two-dimensional image of a medical drug, the reference information and the reference information group also include two-dimensional images" and the like).

In order to attain the above object, a medical drug verification method according to a thirteenth aspect of the present invention includes an exterior information acquiring step of acquiring exterior information on a medical drug, a matching step of performing matching between matching target information based on the acquired exterior information and a reference information group of medical drugs stored in a storage device, a display control step of causing a display device to display, based on a degree of the matching, a plurality of candidates of reference information for a medical drug which is contained in the reference information group and corresponds to the exterior information, an accepting step of accepting selection of the reference information contained in the plurality of candidates, and a reflecting step of reflecting the exterior information and/or the matching target information corresponding to the selected reference information in the stored reference information group, wherein in the reflecting step, the reflection is performed when the degree of the matching for the selected reference information does not satisfy a predetermined criterion.

According to the thirteenth aspect, like the first aspect, the robustness in matching information on a medical drug can be improved. Further, the medical drug verification method according to the thirteenth aspect may further include the same configurations as those of the second to twelfth aspects. A program for causing a medical drug verification device or a computer to execute the medical drug verification methods of these aspects, and a non-transient recording medium on which a computer-readable code of the program is recorded can also be included in an aspect of the present invention.

According to a medical drug verification method of a fourteenth aspect, in the thirteenth aspect, the exterior information, the reference information, and the reference information group include at least one of two-dimensional or three-dimensional shape data and/or exterior data of a medical drug, and a two-dimensional image or a three-dimensional image of a medical drug. Like the twelfth aspect, the fourteenth aspect defines one aspect of the exterior information, the reference information, and the reference information group.

In order to attain the above object, a medical drug verification system according to a fifteenth aspect of the present invention includes an exterior information acquiring device configured to acquire exterior information on a medical drug, a matching device configured to perform matching between matching target information based on the acquired exterior information and a reference information group of medical drugs stored in a storage device, a display control device configured to cause a display device to display, based on a degree of the matching, a plurality of candidates of reference information for a medical drug which is contained in the reference information group and corresponds to the exterior information, an accepting device configured to accept selection of the reference information included in the plurality of candidates, and a reflecting device configured to reflect the exterior information and/or the matching target information corresponding to the selected reference information in the stored reference information group, wherein the reflecting device performs the reflection when the degree of the matching for the selected reference information does not satisfy a predetermined criterion.

According to the fifteenth aspect, like the first aspect, the robustness in matching information on a medical drug can be improved. Further, the medical drug verification system according to the fifteenth aspect may further include the same configurations as those of the second to twelfth aspects.

According to a medical drug verification system of a sixteenth aspect, in the fifteenth aspect, the exterior information, the reference information, and the reference information group include at least one of two-dimensional or three-dimensional shape data and/or exterior data of a medical drug, and a two-dimensional image or a three-dimensional image of a medical drug. Like the twelfth aspect, the sixteenth aspect defines one aspect of the exterior information, the reference information, and the reference information group.

As described above, according to the medical drug verification device, the medical drug verification method, and the medical drug verification system of the present invention, it is possible to enhance robustness in matching information on medical drugs.

### BRIEF DESCRIPTION OF THE DRAWINGS

Figure 1 is a diagram showing a configuration of a medical drug verification device according to a first embodiment.
Figure 2 is a diagram showing how a packaged medical drug is transported.
Figure 3 is a side view showing an arrangement of light sources and cameras.
Figure 4 is a plan view showing the arrangement of the light sources and the cameras.
Figure 5 is a diagram showing a configuration of a processing unit.
Figure 6 is a diagram showing information stored in a storage unit.
Figure 7 is a flowchart showing processing of a medical drug verification method according to the first embodiment.
Figure 8 is a first explanatory diagram used to show switching of an illumination direction.
Figure 9 is a second explanatory diagram used to show the switching of the illumination direction.
Figure 10 is a diagram how a plurality of candidates for a reference image are displayed.
Figure 11 is another diagram showing how the plurality of candidates for the reference image are displayed.
Figure 12 is a diagram showing an example of a matching result in a state where a reference image is added.
Figure 13 is still another diagram showing how the plurality of candidates for the reference image are displayed.
Figure 14 is a diagram showing how the reference image and a reflection date and time are displayed in association with each other.
Figure 15 is a diagram showing how the reference image is being updated.
Figure 16 is a diagram showing a configuration of a medical drug verification device according to a second embodiment.
Figure 17 is a side view showing an arrangement of light sources and cameras when identification of a medical drug is supported.
Figure 18 is a plan view showing the arrangement of the light sources and the cameras when the identification of the medical drug is supported.
Figure 19 is a flowchart showing processing of a medical drug verification method according to a second embodiment.
Figure 20 is a diagram showing how the relationship between a bisection line and a printed character varies.
Figure 21 is another diagram showing how the relationship between the bisection line and the printed character varies.

### DESCRIPTION OF THE PREFERRED EMBODIMENTS

Embodiments of a medical drug verification device, a medical drug verification method, and a medical drug verification system according to the present invention will be described hereinafter in detail with reference to the accompanying drawings. Note that the medical drug verification system according to the present invention includes a plurality of devices each having the function of each component constituting the medical drug verification device described below.

### <First Embodiment>

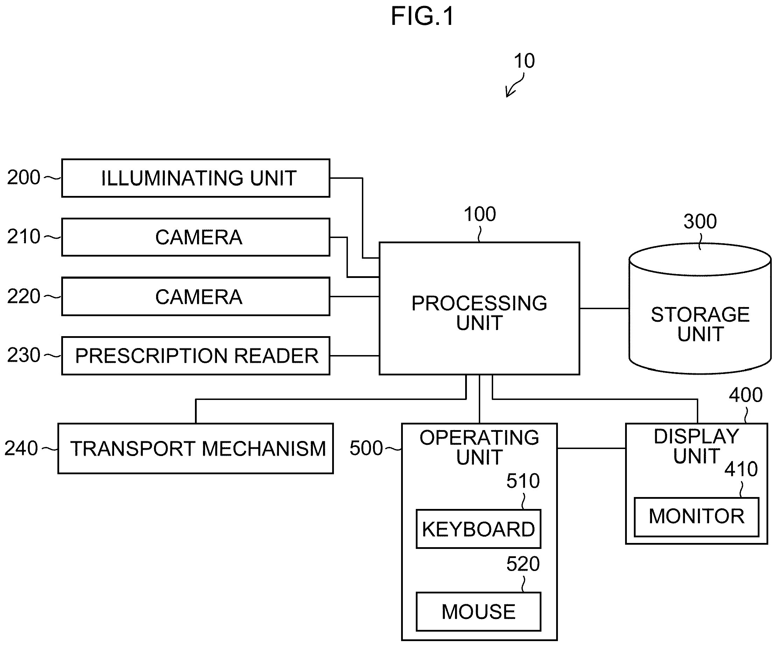

Figure 1 is a diagram showing a configuration of a medical drug verification device 10 (medical drug verification device) according to a first embodiment of the present invention. The medical drug verification device 10 includes a processing unit 100, a storage unit 300 (storage device), a display unit 400 (display device), an operating unit 500, and a transport mechanism 240, and an illuminating unit 200 (light source), a camera 210 (imaging unit), a camera 220 (imaging unit), and a prescription reader 230 are connected to the processing unit 100.

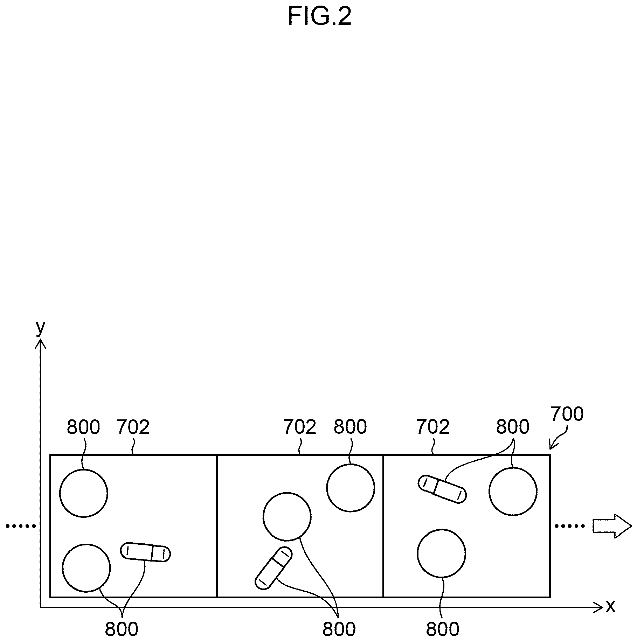

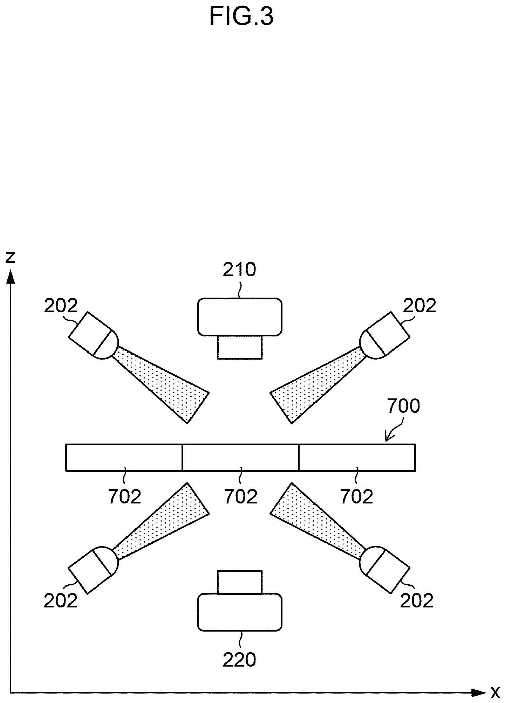

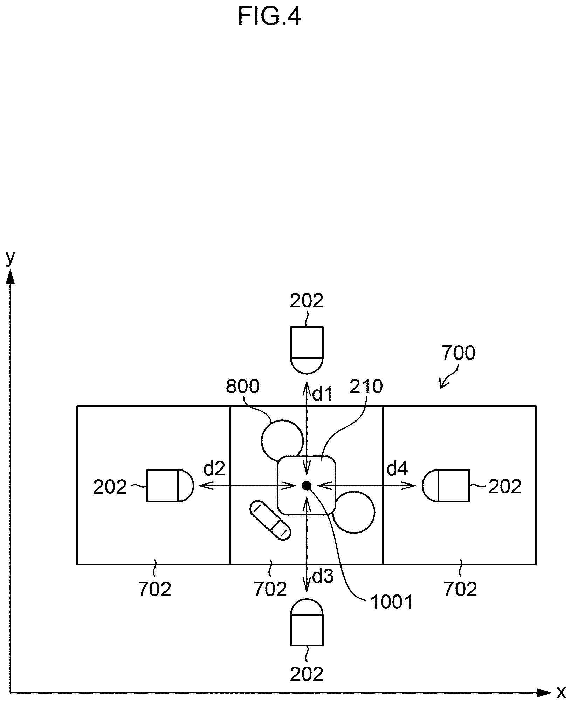

The camera 210 and the camera 220 are configured by digital cameras. As shown in Figure 2, the camera 210 is arranged on a vertically upper side (+Z side of Figure 3) of a dose package belt 700 configured by continuously connecting dose packaging bags 702 (dose packages), and the camera 220 is arranged on a vertically lower side (-Z side of Figure 3) of the dose package belt 700. Medical drugs 800 (tablets, capsules, etc.) packaged in the dose packaging bags 702 are imaged from the upper and lower sides (a plurality of different directions) to acquire images of the front and back surfaces of the medical drugs 800. The dose packaging bags 702 (dose package belt 700) are transported in the +X direction of Figure 2 (an axis along a longitudinal direction of the dose package belt 700; the direction of an arrow of Figure 2) by the transport mechanism 240, and a plurality of light sources 202 (light source) included in the illuminating unit 200 illuminate the dose packaging bags 702 from four directions of the upper and lower sides during imaging. Intervals (d1, d2, d3, d4) between each of the plurality of light sources 202 and an imaging optical axis 1001 of the cameras 210 and 220 are the same. In other words, the plurality of light sources 202 are spaced from the imaging optical axis 1001 at an equal interval (d1 = d2 = d3 = d4) (see Figure 4).

The prescription reader 230 reads prescription information (one aspect of related information). For example, information on a patient's name, prescribed medical drugs, the number or quantity of the medical drugs, etc., is read from a prescription written on paper by OCR (Optical Character Recognition). When a barcode or the like indicating the information on the prescribed medical drugs is recorded on the prescription, the information on the prescribed medical drugs, the number or quantity of the prescribed medical drugs, etc., may be read from the barcode. Further, a user such as a doctor or a pharmacist may read the prescription, and input the prescription information by an input device such as a keyboard 510 and/or the mouse 520 or the like of the operating unit 500.

### <Configuration of Processing Unit>

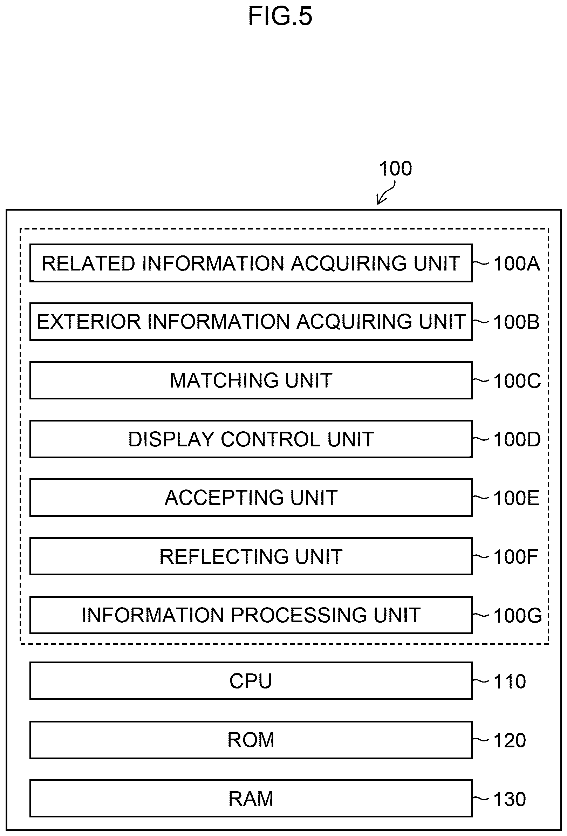

The processing unit 100 verifies medical drugs packaged in a dose packaging bag 702 based on images imaged by the cameras 210 and 220 and information read by the prescription reader 230 or the like. As shown in Figure 5, the processing unit 100 includes a related information acquiring unit 100A (related information acquiring unit), an exterior information acquiring unit 100B (exterior information acquiring unit), a matching unit 100C (matching unit), a display control unit 100D (display control unit), an accepting unit 100E (accepting unit), a reflecting unit 100F (reflecting unit), and an information processing unit 100G (information processing unit). Further, the processing unit 100 includes a CPU 110 (CPU: Central Processing Unit), ROM 120 (ROM: Read Only Memory), and RAM 130 (RAM: Random Access Memory).

The function of the processing unit 100 described above can be implemented by using various processors (processor). The various processors include, for example, a CPU (Central Processing Unit) which is a general-purpose processor for executing software (programs) to implement various functions. Further, the various processors described above also include programmable logic devices (Programmable Logic Device: PLD) which is a processor and whose circuit configurations can be changed after manufacturing, such as GPU (Graphics Processing Unit) which is a specialized processor for image processing and FPGA (Field Programmable Gate Array). Further, the above-mentioned various processors also include a dedicated electric circuit which is a processor having a circuit configuration specially designed for executing specific processing such as ASIC (Application Specific Integrated Circuit).

The function of each unit may be implemented by one processor, or may be implemented by a plurality of processors of the same type or different types (for example, a plurality of FPGAs, or a combination of CPU and FPGA, or a combination of CPU and GPU). Further, a plurality of functions may be implemented by one processor. Examples of a configuration in which a plurality of functions are implemented by one processor includes firstly a mode in which one processor is configured by a combination of one or more CPUs and software and this processor is implemented as a plurality of functions as typified by a computer, and secondly a mode in which a processor for implementing the functions of the entire system with one IC (Integrated Circuit) chip is used as typified by System On Chip (System On Chip: SoC) or the like. As described above, various functions are configured by using one or more of the above-mentioned various processors as a hardware structure. Further, the hardware structures of these various processors are, more specifically, electric circuits (circuitry) in which circuit elements such as semiconductor elements are combined. These electric circuits may be electric circuits that implement the above-mentioned functions by using logical sum, logical product, logical NOT, exclusive logical sum, and logical operations obtained by combining the above logics.

When the above-mentioned processor or an electric circuit executes software (program), a processor (computer)-readable code of the software to be executed is stored in a non-transient recording medium such as ROM 120 (ROM: Read Only Memory), and the processor refers to the software. The software to be stored in the non-transient recording medium includes a program for executing the medical drug verification method according to the present invention. The code may be recorded on a non-transient recording medium such as various magneto-optical recording devices or semiconductor memories instead of ROM. When processing using software is executed, for example, the RAM 130 (RAM Random Access Memory) is used as a temporary storage area, and for example, data stored in EEPROM (Electronically Erasable and Programmable Read Only Memory) (not shown) may be referred to. The CPU 110 presides over the processing to be performed by these processors or electric circuits.

### <Configuration of Storage Unit>

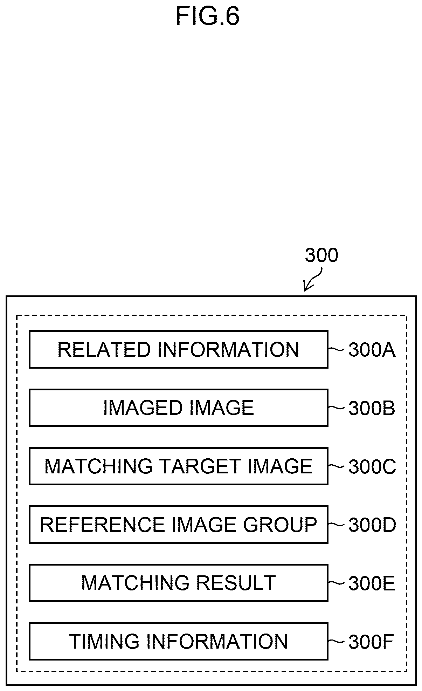

The storage unit 300 is configured by a non-transient recording medium such as a CD (Compact Disk), a DVD (Digital Versatile Disk), a hard disk (Hard Disk) or various semiconductor memories, and a control unit thereof, and information pieces shown in Figure 6 are stored in the storage unit 300 in association with one another. Related information 300A (related information) is information on a prescription read by the prescription reader 230, or information which is input, edited or the like by the user based on the prescription. With respect to the related information 300A, the names of specific medical drugs may be input based on general names of medical drugs described in the prescription, an original medical drug and a generic medical drug may have been mutually changed or the like. Imaged images 300B (image, exterior information) are images of a medical drug which are imaged by the cameras 210 and 220, and include images of the front and back surfaces of the medical drug. When the imaged image includes a plurality of medical drugs, images obtained by extracting a region for a single medical drug from the imaged images may be set as the imaged images 300B. Matching target images 300C (matching target information) are images generated based on the imaged images 300B (for example, images in which an embossment and/or a printed character is emphasized). A reference image group 300D (reference information group) include images of the front and back surfaces of medical drugs, and is an aggregation of reference images (master images) that serve as a reference during template matching. The reference images may include images, etc., provided by pharmaceutical companies, images imaged by means other than the medical drug verification device 10, and images obtained by performing image processing on the above images. A matching result 300E (matching result) is a matching result of a medical drug indicated by an imaged image or a matching target image, and timing information 300F (timing information) is information (date and time of addition, update, etc.) indicating the timing of reflection for the reference information.

### <Configurations of Display Unit and Operating Unit>

The display unit 400 includes a monitor 410 (display device), and can display exterior information on an input image or the like, candidates for a reference image, information stored in the storage unit 300, and the like. The operating unit 500 includes a keyboard 510 and a mouse 520 as input devices and/or pointing devices. The user can perform operations necessary to execute the medical drug verification method according to the present invention, such as an instruction for acquiring exterior information (images, etc.), an instruction for matching medical drugs, selection of reference information, etc., via these devices and the screen of the monitor 410 (described later). Here, the monitor 410 may be configured by a touch panel, and the operations may be performed via the touch panel.

### <Processing of Medical Drug Verification Method>

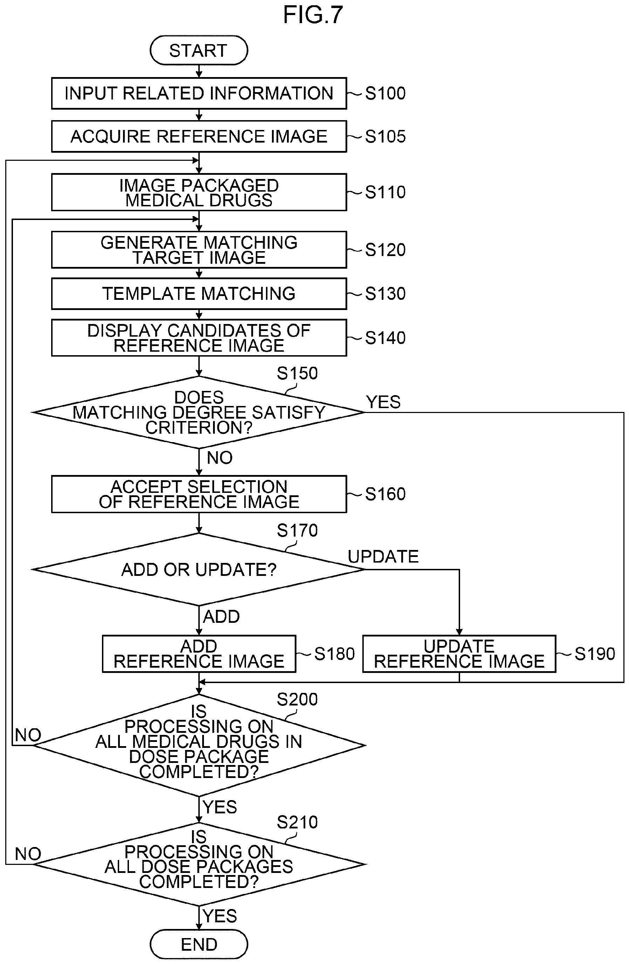

The processing of the medical drug verification method by the medical drug verification device 10 having the above-described configuration will be described with reference to a flowchart of Figure 7. In the following description, a case where the exterior information, the reference information, and the reference information group are images (two-dimensional images) of medical drugs will be described, but as described later, the above-described information may be three-dimensional images or information other than images. Specifically, the exterior information, the reference information, and the reference information group may include at least one of two-dimensional or three-dimensional shape data and/or exterior data of medical drugs, and two-dimensional images and three-dimensional images of the medical drugs. For example, information (image, measurement data, etc.) which can specify the shape, dimension, embossment and/or printed character, color, etc., of the medical drug can be used as "exterior information". Further, information serving as a reference in matching with respect to the shape, dimension, embossment and/or printed character, color, etc., of the medical drug can be set as "reference information", and an aggregation of reference information pieces can be set as "reference information group".

### <Acquisition of Related Information>

The related information acquiring unit 100A acquires related information such as prescription information, for example, via the prescription reader 230 (step S100: related information acquiring step). The related information acquiring unit 100A may acquire input prescription information as related information as it is, or may acquire information which is input or edited via the operating unit 500 based on the prescription by the user. Further, the related information acquiring unit 100A may acquire, as related information, features of medical drugs recognized by visual check or the like by the user (for example, types, shapes, colors, etc., of tablets) or information on names, quantities, dose methods, etc., of medical drugs described in a notebook such as a so-called "medical drug notebook" according to a user's operation, and use them in addition to the prescription information or instead of the prescription information.

### <Acquisition of Reference Image>

The exterior information acquiring unit 100B acquires a reference image (reference image group, reference information group) based on the related information acquired in step S100 (step S105: reference image acquiring step, reference information acquiring step). For example, a reference image for a medical drug included in prescription information is acquired. The reference image to be acquired may be an image imaged by the medical drug verification device 10 or an image obtained by subjecting the imaged image to image processing (which may be performed by information processing unit 100G, for example). Further, the reference image to be acquired may be a reference image obtained by performing "reflection" described later on the above-described image. The exterior information acquiring unit 100B may acquire (read) a reference image (an image included in the reference image group 300D) from the storage unit 300, or acquire it from an external storage device (server, data base or the like on a network). An image acquired from an external storage device may be subjected to image processing, and acquired as a reference image.

### <Imaging of Packaged Medical Drug>

The exterior information acquiring unit 100B controls the cameras 210 and 220 (imaging unit) to acquire imaged images (imaged images 300B, exterior information) obtained by imaging medical drugs 800 (see Figures 2 and 4) packaged in the dose packaging bag 702 in a plurality of different directions (±Z direction in Figures 2 to 4, vertically upward and downward)) (step S110: exterior information acquiring step). At this time, the dose packaging bag 702 is illuminated by the illuminating unit 200 and the light sources 202. The exterior information acquiring unit 100B stores the imaged images as the imaged images 300B in the storage unit 300.

### <Generation of Matching Target Image>

The information processing unit 100G (information processing unit) generates a matching target image based on the imaged images (step S120: exterior information acquiring step, information processing step). The information processing unit 100G can perform image processing such as emphasis of an embossment and/or a printed character, binarization, and inversion, for example. Note that the information processing unit 100G can perform these image processing not only in the generation of the matching target image (one aspect of the matching target information) based on the imaged images, but also in the generation of the reference image (one aspect of the reference information). An example of generating an embossment-emphasized image which is one aspect of image processing will be described below.

### <Generation of Embossment-emphasized Image>

The exterior information acquiring unit 100B sequentially switches an illumination direction for medical drugs by switching a light source for illuminating the medical drugs among the plurality of light sources 202 included in the illuminating unit 200 (see Figures 3 and 4). The exterior information acquiring unit 100B causes the cameras 210 and 220 (imaging units) to image images of the medical drugs each time the illumination direction for the medical drugs is switched. The information processing unit 100G combines a plurality of imaged images (exterior information) corresponding to a plurality of illumination directions to generate an emphasized image (exterior information). According to this aspect, it is possible to accurately recognize an embossment on a medical drug without being affected by the illumination direction in which the medical drug is illuminated, and the surface shape of the medical drug.

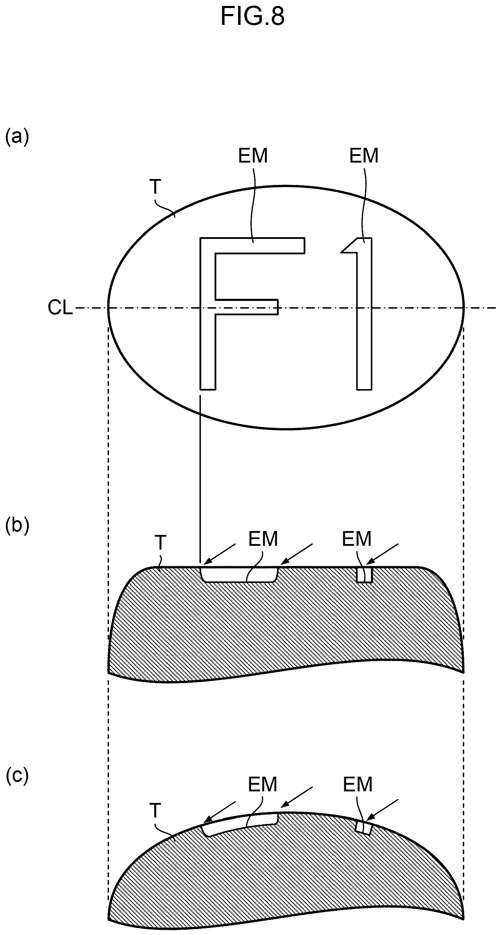

As shown in Figure 8, an embossment EM is configured by grooves formed on the surface of a medical drug T. Portion (a) of Figure 8 is a front view of the embossment EM, portion (b) is a cross-sectional view of the medical drug T which is taken along a straight-line CL in Figure 8, and portion (c) is a cross-sectional view of the medical drug T having a surface shape different from that of the portion (b). When the embossment EM is illuminated from one direction side, shadows are generated along the contours of the embossment EM on the illumination light source side. The directions, shapes, and intensities of the shadows differ according to the illumination direction, the shape of the embossment EM, and the surface shape of the medical drug T.

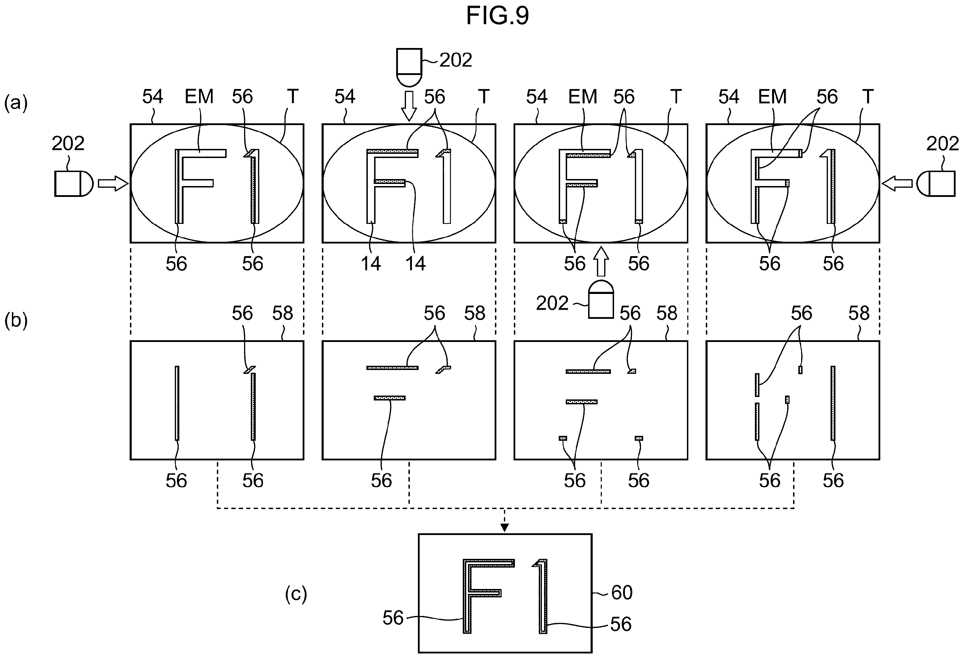

As shown in an upper stage (portion (a)) of Figure 9, a shadow image 56 is generated along the contours of the embossment EM on the illumination light source side (a side of the light source 202 on which illumination light is irradiated) in each of medical drug region image data 54 of four directions. Here, the embossment EM has the same color as the surface of the medical drug T, so that it is difficult to identify the embossment EM on an actual image. However, the embossment EM is illustrated in Figure 9 to be identifiable in order to describe the relationship between the embossment EM and the shadow image 56 according to the illumination direction.

As shown in a middle stage (portion (b)) of Figure 9, the information processing unit 100G performs edge detection processing on each of the medical drug region image data 54 of the four directions for each medical drug T, thereby extracting feature image data 58 corresponding to the shadow image 56 from each medical drug region image data 54. As a result, feature image data 58 for each of the illumination directions of four directions (hereinafter referred to as feature image data 58 in four directions) is obtained for each of the medical drugs T in each dose package, and the information processing unit 100G sequentially stores the feature image data 58 on four directions for the each medical drug T into the storage unit 300.

As shown in a lower stage (portion (c)) of Figure 9, the information processing unit 100G reads out the feature image data 58 on four directions for each medical drug T in one dose package from the storage unit 300, and integrates the feature image data 58 on four directions for each medical drug T to generate integrated image data 60. For example, the feature image data 58 on four directions are superimposed to be integrated. As a result, the shadow images 56 each of which is obtained in each of the four illumination directions are integrated into one shadow image, so that all the contours of the embossment EM become clear on the integrated image data 60. Then, integrated image data 60 (embossment-emphasized image) is generated for each medical drug T in one dose package, and each integrated image data 60 is stored as a reference image (reference image group 300D) or a matching target image (matching target image 300C) in the storage unit 300.

The information processing unit 100G can generate not only an embossment-emphasized image, but also a print-emphasized image by edge emphasis, change of the density or color of a printed portion and/or a background portion, binarization, etc., of imaged images.

### <Template Matching>

The matching unit 100C (matching unit) performs template matching (matching) between the matching target image 300C (matching target information) generated in step S120 and the reference images (reference image group 300D: reference information group) acquired in step S105 (step S130: matching step). The template matching can be performed by a well-known method (for example, a method described in Japanese Patent Application Laid-Open No. 2014-67342), and for example, it is possible to calculate the degree of matching in coincidence, similarity, correlation, etc., between the matching target image 300C and the reference image while relatively moving and/or rotating the matching target image 300C and the reference image. The images may be enlarged or reduced. The matching unit 100C stores a matching result as a matching result 300E in the storage unit 300.

### <Display of Candidates of Reference Image>

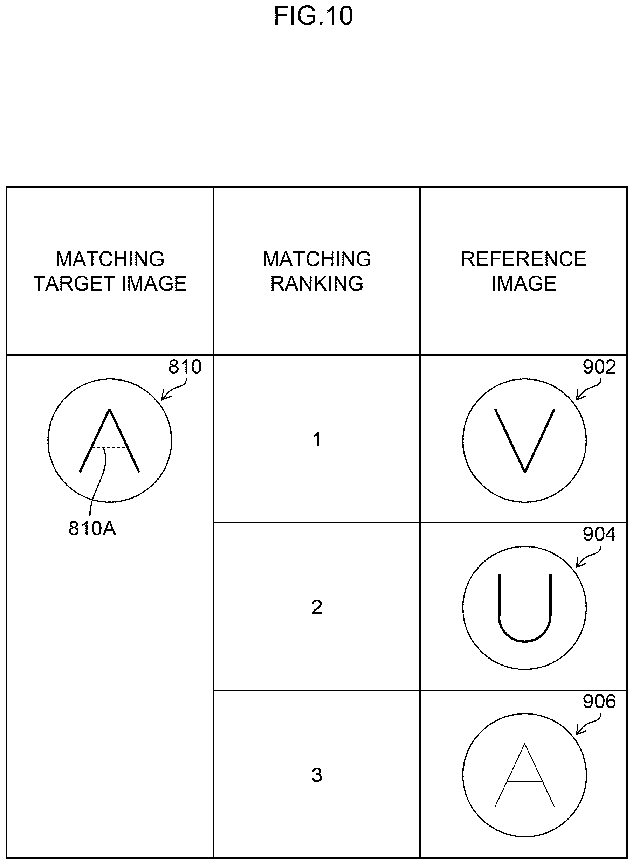

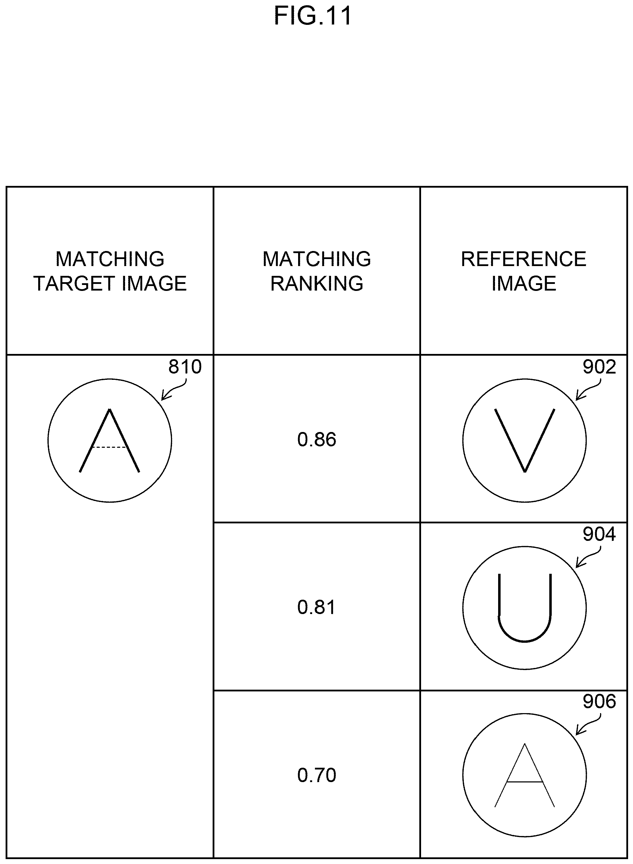

The display control unit 100D (display control unit) displays a plurality of candidates of the reference image (reference information) on a monitor 410 (display device) based on the degree of matching (step S140: display control step). Specifically, the display control unit 100D displays a plurality of candidates of a reference image included in the reference image group 300D (reference information group) for a medical drug corresponding to a matching target image (exterior information). Figure 10 is a diagram showing a display example of the candidates, and displays a matching target image 810 and a plurality of candidates of a reference image (reference images 902, 904, and 906) together with matching ranking (a candidate having a higher matching degree is ranked as higher one). Although only one side of a medical drug is displayed in Figure 10, both sides may be displayed (the same applies to the following figures). Figure 11 is another display example of the candidates, and displays the reference image and the matching degree thereof (similarity and the like; 1.0 is set in the case of perfect matching).

### <Selection of Reference Image>

The reflecting unit 100F (reflecting unit) determines whether the matching degree satisfies a predetermined criterion (step S150: determination step). The following description will be made based on "the matching ranking is 1st and/or the matching degree is 0.80 (an example of a reference value) or more". The reflecting unit 100F (reflecting unit) can set an item of the criterion (rank, degree or the like) and the content thereof (a value of rank, matching degree or the like) by a user's operation via the operating unit 500, and can make a determination by referring to the set criterion. When the criterion is satisfied (for example, the rank is 1st, the matching degree is 0.80 or more, or the like), the processing proceeds to step S200 without reflecting the reference image described later. At this time, the reflecting unit 100F may display a message stating "do not reflect the reference image because matching criterion is satisfied" on the monitor 410. On the other hand, when the criterion is not satisfied, selection of a reference image is accepted, and reflection is performed as follows.

In the examples of Figures 10 and 11, the matching target image 810 should originally most match the reference image 906. However, a horizontal portion of the embossment (reference numeral and character 810A, represented by a dotted chain line) is thin and unclear due to an imaging condition or the like, and the embossment appears to be close to a character V. Therefore, the matching degree thereof with the reference image 902 becomes higher, the matching ranking of the reference image 906 is 3rd (lower than the predetermined ranking (1st)), and the matching degree is 0.79 (which does not satisfy the predetermined reference value (0.80)), so that the above-mentioned criterion is not satisfied (NO in step S150). Therefore, the accepting unit 100E accepts selection of a reference image (reference information) included in the plurality of displayed candidates (reference images 902, 904 and 906 in the examples of Figures 10 and 11) (step S160: accepting step). In the examples of Figures 10 and 11, it is assumed that selection of the reference image 906 (correct image) by the user's operation is accepted via the operating unit 500.

### <Reflection in Reference Information Group>

When a reference image is selected, the reflecting unit 100F reflects the exterior information and/or the matching target information corresponding to the selected reference information in the stored reference information group. Specifically, the matching target image 810 (matching target information) corresponding to the selected reference image 906 (reference information) is reflected in the reference image group 300D (reference information group) stored in the storage unit 300 (storage device). As described below, "reflection" includes addition and update of the reference image, and the reflecting unit 100F can determine whether to add or update the reference image based on the user's setting via the operating unit 500 (step S170: determination step).

### <When Reference Image Is Added>

The reflecting unit 100F additionally stores, into the storage unit 300 (storage device), the imaged image (exterior information) and/or the matching target image 810 (matching target information) corresponding to the selected reference image 906 (reference information) as a new reference image (an image constituting the reference image group 300D, reference information) for a medical drug designated by the matching target image 810 (hereinafter referred to as a "medical drug A") (step S180: reflecting step, adding step). This "addition" increases the number of reference images for the medical drug A. The reflecting unit 100F can store information indicating the timing of addition (reflection) as timing information 300F in the storage unit 300 in association with a reference image 908 (reference information).

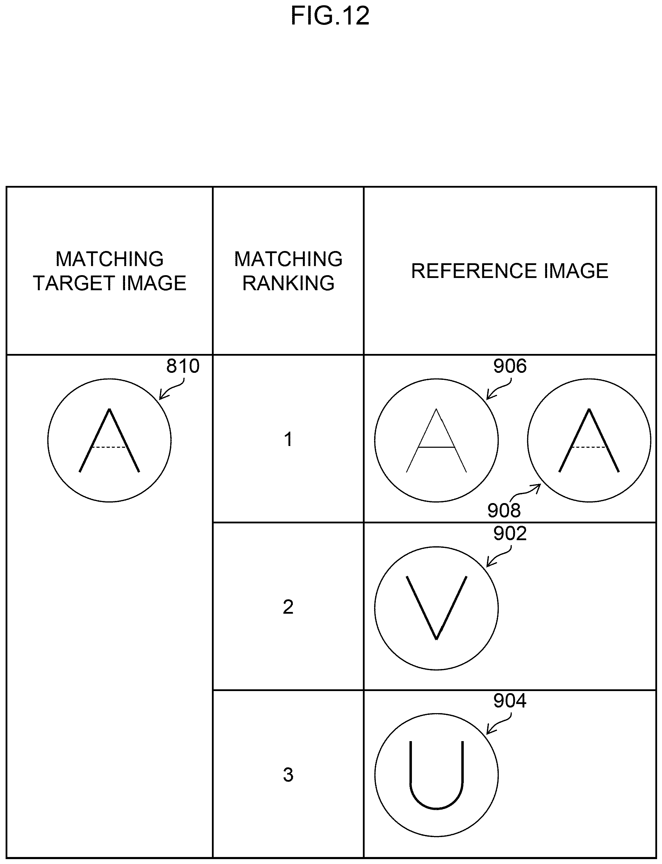

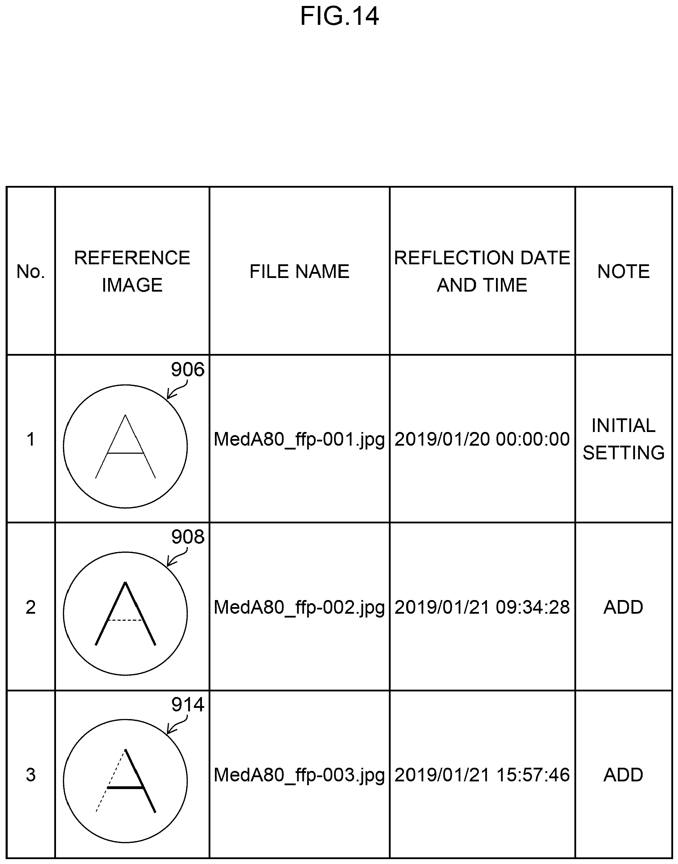

Figure 12 is a diagram showing an example of a matching result in a state where the matching target image 810 is added as the reference image 908 for the medical drug A by the above-described processing. Figure 12 shows a state where matching is performed with the reference image 908 being added, and the matching target image 810 (the same as the examples of Figures 10 and 11) matches the added reference image 908, so that the matching ranking of the medical drug A becomes the 1st (correctly matched state). In this way, when the degree of matching does not meet a predetermined criterion, an appropriate reference image (a plurality of reference images having various reflections for the embossment: for example, see Figure 14) is being added by adding a reference image (reflection of the reference information). As a result, accurate matching can be performed on various matching target images (one aspect of matching target information) in which differences in imaging condition and individual differences in medical drug exist, so that robustness in matching information on medical drugs can be improved.

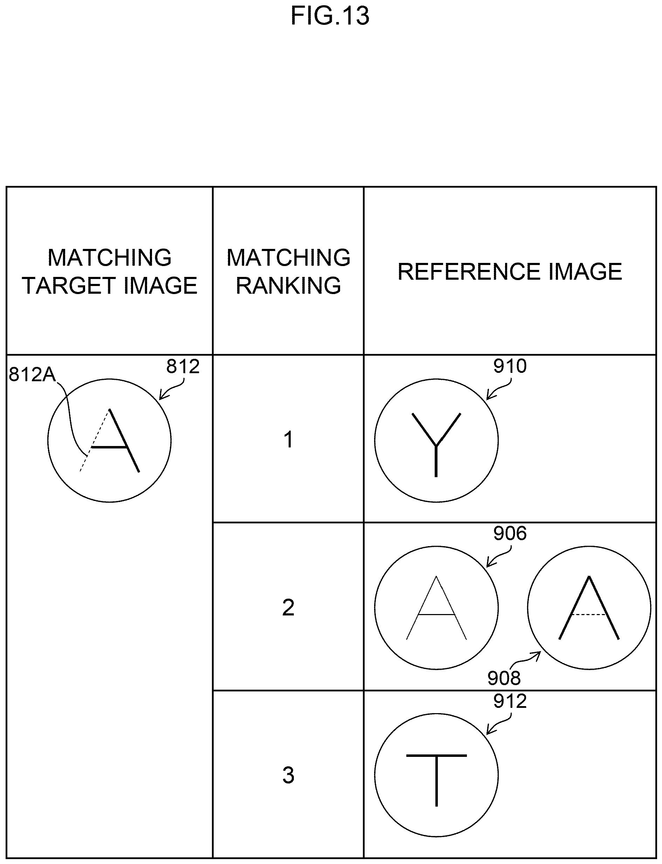

Figure 13 is a diagram showing another example of the matching result. In this example, a left side of an embossment of a matching target image 812 (reference numeral and character 812A, represented by a dotted chain line) is thin and unclear, and the embossment appears to be close to a character Y. Therefore, the matching degree with a reference image 910 is higher. Even in such a case, it is possible to accept the above-mentioned selection and add a reference image.

Figure 14 is a diagram showing how reference images are added. The display control unit 100D can display, on the monitor 410 (display device), a reference image (reference information), a file name, and timing information regarding the reference image (reflection date and time in an example of Figure 14) while associating the reference image, the file name and the timing information with one another, for example, according to a user's instruction via the operating unit 500.

### <When Reference Image Is Updated>

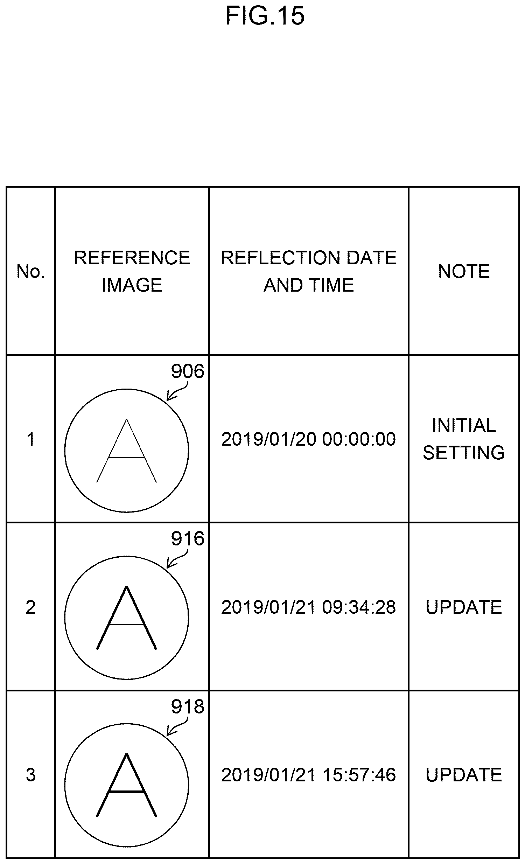

When the reference image is updated in the examples of Figures 10 and 11 (when the determination result in step S170 is "update"), the reflecting unit 100F generates a new reference image (reference information) based on the imaged image (exterior information) corresponding to the selected reference image 906 (reference information) and/or the matching target image 810 (matching target information) and the reference image 906 stored in the storage unit 300. The reflecting unit 100F updates the stored reference image 906 with the generated master image (reference image) (step S190: reflecting step, updating step). In the case of updating, the number of reference images is maintained. The reflecting unit 100F can generate a new reference image, for example, by weighting and adding a plurality of images. Figure 15 is a diagram showing how the reference image is updated. Initially, the embossment of the reference image 906 is thin and unclear, but after first updating, the embossment of the reference image 916 is thick and clear at right and left portions thereof. Further, after second updating, the embossment of the reference image 918 is also thick and clear at a horizontal portion thereof. As in the case of addition of the reference image, the reflecting unit 100F can store information indicating the timing of update (reflection) as timing information 300F into the storage unit 300 in association with the reference image 908 (reference information), and the display control unit 100D can display the reference image and the timing information on the monitor 410 while the reference image and the timing information are associated with each other.

The update of the reference image as described above also makes it possible to use an appropriate reference image (a reference image having a thick and clear embossment in the example of Figure 15), and improve the robustness in matching information on medical drugs.

### <Second Embodiment>

### <Medical Drug Identification Support>

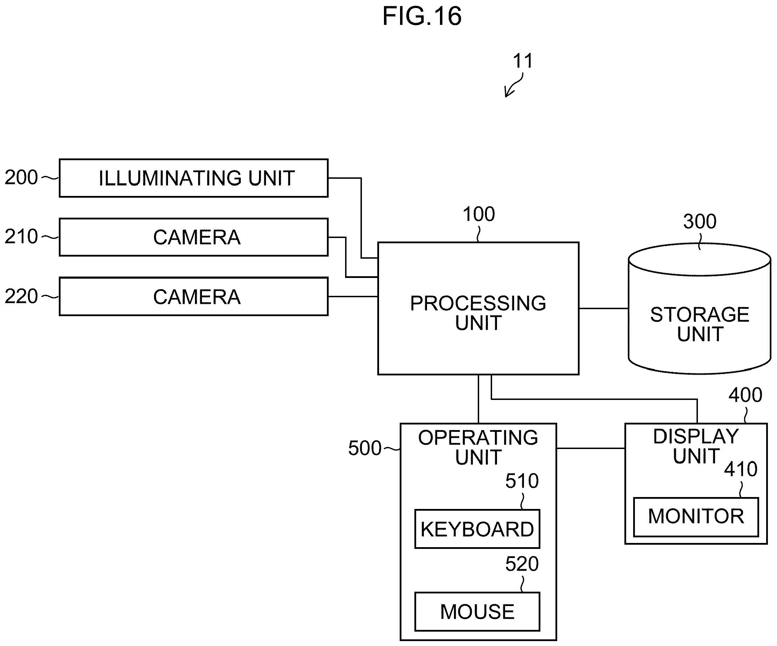

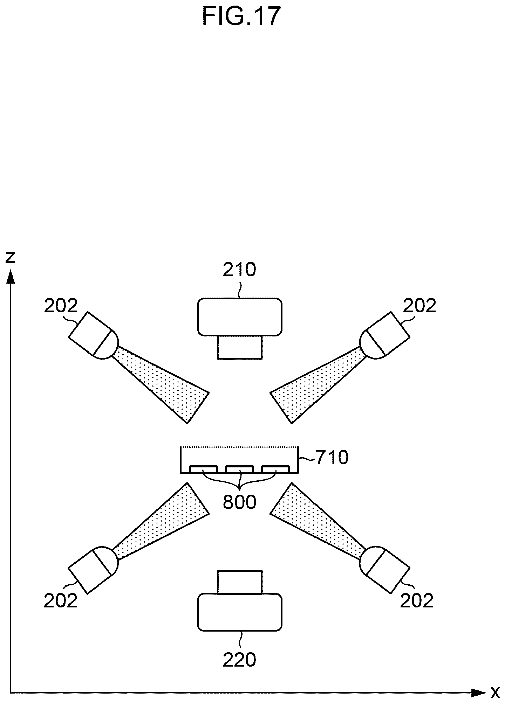

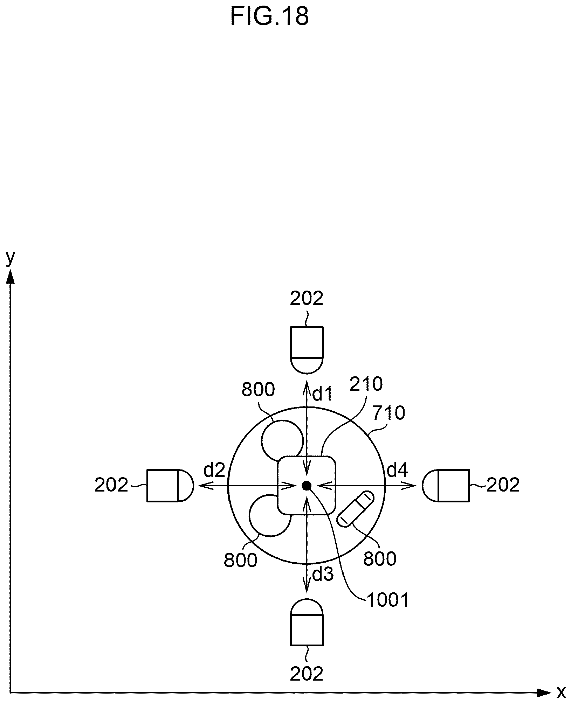

In the above-described embodiment, the matching of packaged medical drugs which is mainly performed in medical drug audit support has been described, but the medical drug verification device, the medical drug verification method and the medical drug verification system of the present invention can also be applied to identification support for medical drugs which are brought to hospitals, pharmacies and the like by patients. Figure 16 is a diagram showing a configuration of a medical drug verification device 11 for supporting identification of medical drugs in which the prescription reader 230 and the transport mechanism 240 for a dose package belt are omitted as compared with the medical drug verification device 10 according to the first embodiment. When the medical drug verification device 11 is used to perform identification support, as shown in a side view of Figure 17, medical drugs 800 are placed in a container 710 such as a petri dish instead of a dose packaging bag, and imaged. Figure 18 is a plan view of the state shown in Figure 17. Other configurations to perform the identification are the same as those of the medical drug verification device 10 described above. The matching processing for medical drugs by the medical drug verification device 11 can be performed in the same manner as in the case of the medical drug verification device 10, but when medical drugs brought by patients are identified, it may be impossible to confirm a prescription. In this case, the related information acquiring unit 100A receives, as related information, features of medical drugs recognized by visual check or the like (for example, the types, shapes, colors, etc., of tablets), or names, quantities, dose methods, etc., of medical drugs described in a notebook such as a so-called "medical drug notebook" according to a user's operation, and use them instead of prescription data.

### <Processing When Identification Support Is Performed>

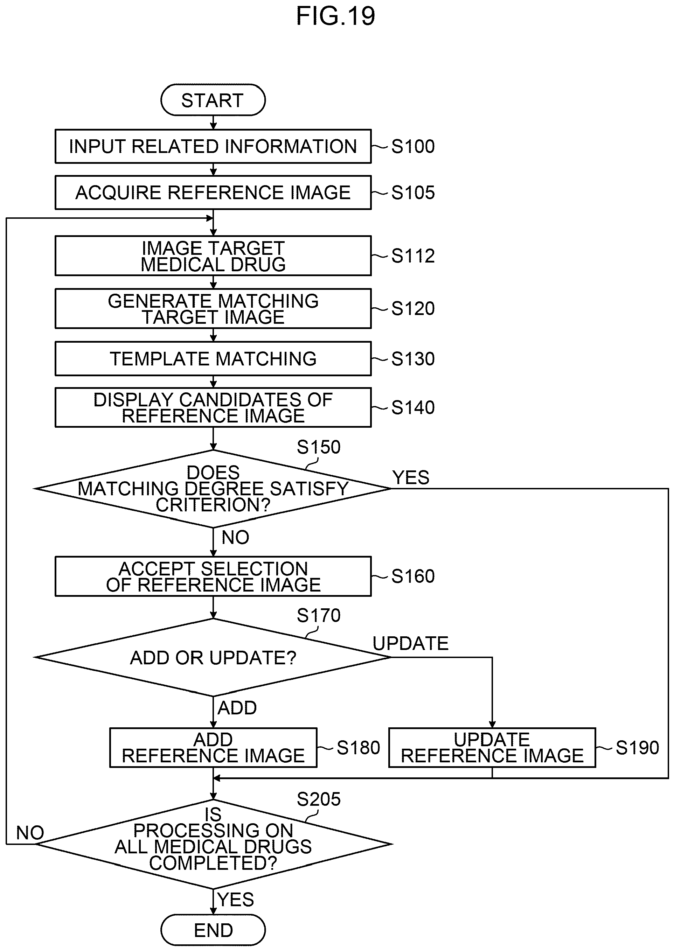

Figure 19 is a flowchart of medical drug verification processing (identification support processing) by the medical drug verification device 11. In Figure 19, imaging of a target medical drug can be performed in step S112 (exterior information acquiring step) instead of step S110 in Figure 7. In connection with this processing, step S210 in Figure 7 is omitted, and step S200 is changed to step S205. Other processing (matching, display of candidates, acceptance of selection, reflection, etc.) are the same as those of the flowchart of Figure 7. Such processing enables the robustness in matching information on medical drugs to be also improved by the medical drug verification device, the medical drug verification method and the medical drug verification system according to the present invention for the medical drug identification support.

### <Other Examples of Exterior Information, Reference Information, etc.>

In the first and second embodiments described above, the case where the exterior information, the reference information, and the reference information group are images of medical drugs (two-dimensional images) has been described. However, in the present invention, the exterior information, the reference information, and the reference information group of the medical drugs may not be two-dimensional images, but other information with which shapes, dimensions, colors, embossments and/or printed characters, etc., can be grasped (shape data and/or exterior data, three-dimensional images (stereo images) of medical drugs) can be used. Specifically, the exterior information, the reference information, and the reference information group of medical drugs may include at least one of two-dimensional or three-dimensional shape data and/or exterior data of medical drugs, and two-dimensional images or three-dimensional images of the medical drugs. Hereinafter, other examples of information (measurement data) which can be used as the exterior information, the reference information, and the reference information group will be described.

When three-dimensional data of a medical drug is acquired by an optical cutting method, the medical drug is irradiated with a line-shaped laser beam from a laser light source, reflected light is imaged by a camera, and an image is analyzed to calculate a cross-sectional shape at a position where the laser beam is irradiated. This processing is repeated while moving the medical drug and the laser beam relatively (for example, while moving them in a direction orthogonal to a cut section), whereby the three-dimensional shape, dimension, embossment, etc., of the medical drug can be measured (restored). During measurement, the cut section may be moved by changing the irradiation direction of the laser beam with one or more rotatable mirrors.

Examples of a three-dimensional measurement method other than the optical cutting method include a pattern projection method, a TOF method (TOF: Time of Flight), a stereo method, and an SfM method (SfM: Solid from Motion), etc. The pattern projection method is a method in which a striped pattern is projected onto a measurement target object with a laser beam or the like, the state of the stripe is imaged by a stereo camera (a plurality of cameras arranged to be spaced from each other at a distance), and a three-dimensional shape is restored based on the obtained image. Likewise, the stereo method using a three-dimensional image acquired by a stereo camera can also be used. In the TOF method, the distance to a measurement target point is determined based on a period of time from irradiation of the measurement target object with a laser beam till reception of reflected light, and the measurement is repeated while changing the target point, thereby obtaining a three-dimensional shape.

In the SfM method, a plurality of images of a measurement target object are acquired, and a camera position during imaging is determined based on feature points in the acquired images. When the camera position is determined, the three-dimensional arrangement of the feature points is also restored. Further, by using a method called MVS (Multi-View Stereo) in addition to the SfM method, a dense point cloud can be restored. Further, by using a code target and a positioning target together, the measurement can be performed with higher accuracy.

In the medical drug verification device, the medical drug verification method, and the medical drug verification system of the present invention, the measurement data obtained by the above-exemplified methods can be used as exterior information, reference information, and reference information group, whereby it is possible to improve the robustness in matching the information on medical drugs as in the case of the first and second embodiments. Further, the medical drug verification device of the present invention may be provided with a measuring unit (laser light source, pattern projection device, stereo camera, restoration processing unit or the like) using these methods, and the medical drug verification method of the present invention may include a measuring step using these methods.

The embodiments and other aspects of the present invention have been described above. However, the present invention is not limited to the above-described aspects, and various modifications can be made without departing from the spirit of the present invention.

### Explanation of References

- 10: medical drug verification device
- 11: medical drug verification device
- 54: medical drug region image data
- 56: shadow image
- 58: feature image data
- 60: integrated image data
- 100: processing unit
- 100A: related information acquiring unit
- 100B: exterior information acquiring unit
- 100C: matching unit
- 100D: display control unit
- 100E: accepting unit
- 100F: reflecting unit
- 100G: information processing unit
- 110: CPU
- 120: ROM
- 130: RAM
- 200: illuminating unit
- 202: light source
- 210: camera
- 220: camera
- 230: prescription reader
- 240: transport mechanism
- 300: storage unit
- 300A: related information
- 300B: imaged image
- 300C: matching target image
- 300D: reference image group
- 300E: matching result
- 300F: timing information
- 400: display unit
- 410: monitor
- 500: operating unit
- 510: keyboard
- 520: mouse
- 700: dose package belt
- 702: dose packaging bag
- 710: container
- 800: medical drug
- 810: matching target image
- 812: matching target image
- 850: laser light source
- 852: medical drug
- 854: camera
- 856: mirror
- 902: reference image
- 904: reference image
- 906: reference image
- 908: reference image
- 910: reference image
- 916: reference image
- 918: reference image
- 1001: imaging optical axis
- EM: embossment
- H: height
- S100-S210: each step of medical drug verification method
- T: medical drug
- W: width

## Claims

1. A medical drug verification device comprising:
an exterior information acquiring unit configured to acquire exterior information on a medical drug;
a matching unit configured to perform matching between matching target information based on the acquired exterior information and a reference information group of medical drugs stored in a storage device;
a display control unit configured to cause a display device to display, based on a degree of the matching, a plurality of candidates of reference information for a medical drug which is contained in the reference information group and corresponds to the exterior information;
an accepting unit configured to accept selection of the reference information contained in the plurality of candidates; and
a reflecting unit configured to reflect the exterior information and/or the matching target information corresponding to the selected reference information in the stored reference information group,
wherein the reflecting unit performs the reflection when the degree of the matching for the selected reference information does not satisfy a predetermined criterion.

2. The medical drug verification device according to claim 1, wherein the reflecting unit additionally stores, in the storage device, the exterior information and/or the matching target information corresponding to the selected reference information as new reference information for a medical drug designated by the exterior information, thereby performing the reflection.

3. The medical drug verification device according to claim 1, wherein the reflecting unit generates new reference information based on the exterior information and/or the matching target information corresponding to the selected reference information and the stored reference information, and updates the stored reference information with the generated reference information, thereby performing the reflection.

4. The medical drug verification device according to any one of claims 1 to 3, wherein when the degree for the selected reference information does not satisfy a predetermined reference value, the reflecting unit determines that the degree does not satisfy the criterion, and performs the reflection.

5. The medical drug verification device according to any one of claims 1 to 4, wherein when ranking determined by the degree for the selected reference information is lower than predetermined ranking, the reflecting unit determines that the degree does not satisfy the criterion and performs the reflection.

6. The medical drug verification device according to any one of claims 1 to 5, further comprising an information processing unit configured to subject the exterior information to processing for emphasizing an embossment and/or a printed character of a medical drug, and the matching unit performs the matching by using the exterior information which has been subjected to the processing.

7. The medical drug verification device according to any one of claims 1 to 6, wherein the reflecting unit stores the reference information in the storage unit in association with timing information indicating a timing of the reflection.

8. The medical drug verification device according to claim 7, wherein the display control unit causes the display device to display the reference information and the timing information in association with each other.

9. The medical drug verification device according to any one of claims 1 to 8, further comprising an imaging unit configured to image the medical drug, wherein the exterior information acquiring unit acquires an image imaged by the imaging unit.

10. The medical drug verification device according to any one of claims 1 to 9, further comprising a light source configured to illuminate the medical drug.

11. The medical drug verification device according to any one of claims 1 to 10, further comprising a related information acquiring unit configured to acquire related information on a medical drug, wherein the matching unit performs the matching by using the reference information for a medical drug specified by the related information.

12. The medical drug verification device according to any one of claims 1 to 11, wherein the exterior information, the reference information, and the reference information group include at least one of two-dimensional or three-dimensional shape data and/or exterior data of a medical drug, and a two-dimensional image or three-dimensional image of a medical drug.

13. A medical drug verification method comprising:
an exterior information acquiring step of acquiring exterior information on a medical drug;
a matching step of performing matching between matching target information based on the acquired exterior information and a reference information group of medical drugs stored in a storage device;
a display control step of causing a display device to display, based on a degree of the matching, a plurality of candidates of reference information for a medical drug which is contained in the reference information group and corresponds to the exterior information;
an accepting step of accepting selection of the reference information contained in the plurality of candidates; and
a reflecting step of reflecting the exterior information and/or the matching target information corresponding to the selected reference information in the stored reference information group,
wherein in the reflecting step, the reflection is performed when the degree of the matching for the selected reference information does not satisfy a predetermined criterion.

14. The medical drug verification method according to claim 13, wherein the exterior information, the reference information, and the reference information group include at least one of two-dimensional or three-dimensional shape data and/or exterior data of a medical drug, and a two-dimensional image or a three-dimensional image of a medical drug.

15. A medical drug verification system comprising:
an exterior information acquiring device configured to acquire exterior information on a medical drug;
a matching device configured to perform matching between matching target information based on the acquired exterior information and a reference information group of medical drugs stored in a storage device;
a display control device configured to cause a display device to display, based on a degree of the matching, a plurality of candidates of reference information for a medical drug which is contained in the reference information group and corresponds to the exterior information;
an accepting device configured to accept selection of the reference information included in the plurality of candidates; and
a reflecting device configured to reflect the exterior information and/or the matching target information corresponding to the selected reference information in the stored reference information group,
wherein the reflecting device performs the reflection when the degree of the matching for the selected reference information does not satisfy a predetermined criterion.

16. The medical drug verification system according to claim 15, wherein the exterior information, the reference information, and the reference information group include at least one of two-dimensional or three-dimensional shape data and/or exterior data of a medical drug, and a two-dimensional image or a three-dimensional image of a medical drug.
